(19) Europäisches Patentamt
European Patent Office
Office européen des brevets

(11) **EP 1 819 400 B1**

(12) **FASCICULE DE BREVET EUROPEEN**

(45) Date de publication et mention
de la délivrance du brevet:
**12.01.2011 Bulletin 2011/02**

(21) Numéro de dépôt: **05804631.9**

(22) Date de dépôt: **21.11.2005**

(51) Int Cl.:
*A61Q 3/02* *(2006.01)*          *A61K 8/34* *(2006.01)*
*A61K 8/37* *(2006.01)*          *A61K 8/97* *(2006.01)*

(86) Numéro de dépôt international:
**PCT/EP2005/056098**

(87) Numéro de publication internationale:
**WO 2006/056558 (01.06.2006 Gazette 2006/22)**

(54) **COMPOSITION DE VERNIS A BASE DE SOLVANT EXCLUSIVEMENT D'ORIGINE VEGETALE**

LACKZUSAMMENSETZUNG AUF BASIS EINES REIN PFLANZLICHEN LÖSUNGSMITTELS

VARNISH COMPOSITION BASED ON A SOLVENT EXCLUSIVELY OF VEGETABLE ORIGIN

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **23.11.2004 FR 0412409**

(43) Date de publication de la demande:
**22.08.2007 Bulletin 2007/34**

(73) Titulaire: **Durlin France**
**24000 Bergerac (FR)**

(72) Inventeurs:
• **DESWARTVAEGHER, Alain**
**F-64400 Bidos (FR)**
• **FORESTIER, Bernard**
**F-24520 SAINT NEXANS (FR)**
• **MIARD, Sophie**
**F-24520 SAINT GERMAIN ET MONS (FR)**
• **SENET, Jean-Pierre**
**F-77760 BUTHIERS (FR)**
• **THIEBAUD-ROUX, Sophie**
**F-31240 L'UNION (FR)**
• **CRISTEA, Daniela**
**F-78560 Le Port Marly (FR)**
• **DE CARO, Pascale**
**F-31400 TOULOUSE (FR)**
• **GIACINTI, Géraldine**
**F-31470 FONSORBES (FR)**
• **BANDRES, Mathieu**
**F-31400 TOULOUSE (FR)**

(74) Mandataire: **Ahner, Francis et al**
**Cabinet Régimbeau**
**20, rue de Chazelles**
**75847 Paris Cedex 17 (FR)**

(56) Documents cités:
EP-A- 0 191 292          EP-A- 0 898 958
FR-A- 1 267 528          FR-A- 1 366 742
FR-A- 1 390 184          FR-A- 2 676 647
GB-A- 1 433 049          US-A- 5 639 447
US-A-2004/0 185 018

• DATABASE WPI Section Ch, Week 200205
Derwent Publications Ltd., London, GB; Class
E17, AN 2002-039929 XP002336956 & RU 2 174
974 C1 (VORON TECHN ACAD) 20 octobre 2001
(2001-10-20) cité dans la demande
• DATABASE WPI Section Ch, Week 200314
Derwent Publications Ltd., London, GB; Class
D21, AN 2003-146514 XP002336957 & RU 2 194
492 C1 (CHITA MED ACAD) 20 décembre 2002
(2002-12-20) cité dans la demande

EP 1 819 400 B1

**Description**

La présente invention a pour objet une composition de vernis à usage cosmétique ou pharmaceutique réalisée à partir de solvants d'origine naturelle. Ces solvants sont des acétates préparés à partir d'une même molécule naturelle et ont une tension de vapeur inférieure aux acétates d'éthyle, de propyle ou de butyle. Cette propriété induit une moindre émission de Composés Organiques Volatils (COV).

Un arrêté ministériel du 1er mars 1993 considère comme composés organiques volatiles (COV) tout composé qui, à l'exclusion du méthane, contient du carbone et de l'hydrogène (lequel peut être substitué par d'autres atomes comme les halogènes, l'oxygène, le soufre, l'azote ou le phosphore, à l'exception des oxydes de carbone et des carbonates) et qui se trouve à l'état de gaz ou de vapeur dans des conditions normales de température et de pression.

La directive européenne 1999/13/CE complète cette définition et ajoute que tout produit organique ayant une pression de vapeur supérieure à 10 Pa (environ 0,075 mmHg) est considérée comme COV.

Aux Etats-Unis, le seuil de tension de vapeur retenu est bien plus faible, à savoir 0,13 Pa (environ 0,001 mmHg) dans les conditions normales. En Australie, les COV sont définis comme des composés chimiques ayant une pression de vapeur supérieure à 27 Pa à 25°C.

Parmi les COV, on peut notamment citer sans que cette liste soit exhaustive, les solvants, les dégraissants, les dissolvants, les conservateurs, les agents de nettoyage et les disperseurs. Les législations, bien que variables en fonction des pays, tendent toutes vers une réduction de l'émission de ces COV.

En France, la réglementation a été modifiée afin de prendre en compte les exigences issues de la directive européenne 1999/13/CE du 11 mars 1999 relative à la réduction des émissions de COV dues à l'utilisation de solvants organiques dans certaines activités et installations.

En conséquence, il est indispensable de trouver des compositions cosmétiques qui présentent des taux de COV limités.

Les molécules présentées dans le tableau 1 ont une tension de vapeur sensiblement inférieure aux tensions de vapeur des solvants habituellement utilisés dans les vernis à ongles, l'acétate d'éthyle et l'acétate de butyle. L'état de la technique antérieure peut notamment être illustré par les brevets FR-A-1390184 et FR-A-1366742.

Pour évaluer de façon claire et rigoureuse la réduction d'émission de COV entraînée par l'utilisation de nouveaux solvants d'origine naturelle, l'étude a été basée sur un modèle publié en 2002 par l'ADEME dans le rapport intitulé "Les émissions de COV dans le secteur de la production des peintures, vernis, encres d'imprimerie, colles et adhésifs". Le modèle utilisé, le modèle de Clements, permet de calculer les émissions de COV engendrées par l'évaporation de surface lors de la production d'un vernis dans une cuve ouverte.

Les émissions de chaque solvant présent dans une composition de vernis sont calculées par l'équation:

$$Ei = \frac{Mi.Ki.A.Pi.3600.hr}{R.T} .N$$

où:

Ei est l'émission de l'espèce i en kg par an,
Mi est la masse moléculaire de l'espèce i en g/mole,
Ki est le coefficient de transfert de masse de l'espèce i en m.s$^{-1}$ de la phase liquide à la phase gazeuse,
Pi est la pression de vapeur de l'espèce i en kPa,
Hr est la durée en heures de l'opération de fabrication,
A est la surface libre de la cuve en m$^2$,
R est la constante des gaz parfaits (8.314 J.K$^{-1}$.mol$^{-1}$),
T est la température absolue en Kelvin, et
N est le nombre d'opérations de fabrication identiques dans l'année.

Le coefficient de transfert de l'espèce i à la phase gazeuse (Ki) peut être déterminé par l'équation:

$$Ki = 0.00250 \cdot V^{0.78} \cdot \left(\frac{18}{Mi}\right)^{1/3}$$

où:

**Ki** est exprimé en m.s$^{-1}$

**V** est la vitesse de l'air au dessus de la cuve en m.s$^{-1}$

**Mi** est la masse molaire de l'espèce i en g.mol$^{-1}$.

Tableau 1:

| | | |
|---|---|---|
| Comparaison des tensions de vapeur et des points éclairs des solvants habituels pour vernis à ongles (acétates d'éthyle et de butyle) avec des solvants d'origine naturelle. | | |
| | Tension de vapeur à 25°C (Pa) | Point éclair (°C) |
| Acétate d'éthyle | 10300 | - 4 |
| Acétate de butyle | 1990 | 24 |
| Ethanol | 7827 | 12,8 |
| Acétate d'isoamyle | 757 | 25 |
| Alcool isoamylique | 555 | 45,6 |
| Isoamyl carbonate | 374 | 55,5 |
| Ethyl isoamyl éther | 3413 | 9 |
| Méthyl isovalérate | 2426 | 19,4 |
| Ethyl isovalérate | 1047 | 26,7 |

Le terme d'huile de fusel, d'origine allemande, se traduit par "alcool inférieur ou de mauvaise qualité". Aujourd'hui, ce terme désigne l'ensemble des alcools supérieurs, obtenus à divers stades de la fermentation. Les composés de l'huile de fusel peuvent être classifiés en deux grands groupes (PATIL A.G.S.M et al. International Sugar Journal, (2002), 104, 51-54, 56-58):

- la fraction à haut point d'ébullition (HBF - *High Boiling Fraction*), Téb > 132°C. Elle représente seulement 1 à 5% de l'huile de fusel. Les constituants de cette fraction peuvent être classifiés dans trois groupes (SHORUIGIN, P. Pet al. Ber. (1933). 66B: 1087-1093 ; SHORUIGIN, PP et al., Zhurnal Obshchei Khimii (1934), 4 372-394)

  o composés acides (10-25%): alcools supérieurs (hexanol, heptanol, octanol, nonanol), acides gras (butyrique, valérique, caproïque, caprylique, pélagonique, caprique, laurique, myristique, palmitique) et leurs esters (acétates, butyrates),
  o composés basiques (5-10%): di, tri et tétra-méthylpyrazines, et
  o composés neutres (60-80%): terpènes;

- la fraction à faible point d'ébullition (LBF - *Low Boiling Fraction*), T$_{éb}$ < 132°C. Cette fraction représente la partie majoritaire de l'huile de fusel (95-98%) (PATIL déjà cité).

Le tableau 2 ci-après présente quelques compositions de la fraction LBF d'huile de fusel.

Tableau 2:

| | H$_2$O | EtOH | PrOH | Iso- PrOH | BuOH | Iso-BuOH | AmOH actif | Iso-AmOH |
|---|---|---|---|---|---|---|---|---|
| Composition de la fraction LBF des huiles de fusel de différentes origines (%) | | | | | | | | |
| Mêlasse de betterave | - | 10,0 | 0,6 | - | 2,0 | 0,2 | 3,0 | 73,0 |
| Mêlasse de betterave (KUCUK Z. et al. Turkish Journal of Chemistry (1998) 22(3), 289-300. | - | 12,4 | 3,5 | - | - | 9,5 | - | 74,6 |

(suite)

| | H$_2$O | EtOH | PrOH | Iso- PrOH | BuOH | Iso-BuOH | AmOH actif | Iso-AmOH |
|---|---|---|---|---|---|---|---|---|
| Mêlasse de canne à sucre (KHEDR, M.A. et al. Pakistan Journal of Scientific and Industrial Research (1994) 37 (11)488-490. | - | 3,96 | - | | 9,61 | 5,28 | - | 76.86 |
| Mêlasse (ULLMAN'S 1981) | - | nd | 13,2 | - | 0,2-0,7 | 15,8 | 18,4 | 37,4 |
| Pommes de terre (ULLMAN'S 1981) | - | nd | 14,0 | - | 0,5 | 15,5 | 15,0 | 55,0 |
| Fruits (ULLMAN'S 1981) | - | nd | 8,0 | - | 2,0 | 19,0 | 14,0 | 57,0 |
| Céréales (ULLMAN'S 1981) | - | nd | 9,1 | - | 0,2-0,7 | 19,0 | 20,0 | 13,0 |
| Déchets (ULLMAN'S 1981) | - | nd | 7,0 | - | - | 22,0 | 13,0 | 55,0 |

Les pourcentages de chaque alcool varient fortement en fonction de la matière première utilisée pour la fermentation, mais aussi en fonction de la méthode de fermentation ou de distillation.

L'huile de fusel est un liquide de couleur jaune paille à rouge foncé, relativement visqueux, ayant une odeur désagréable. Avant la mise au point de procédés de synthèse chimique, l'huile de fusel était la seule source commerciale d'alcools amyliques.

Suite à la production de 1 000 1 d'alcool, on peut obtenir entre 1 et 11 1 d'huile de fusel. Ce pourcentage dépend de la matière première utilisée et des conditions de fermentation et de distillation, comme illustré dans le tableau 3 qui suit.

Tableau 3:

| Rendements d'obtention de l'huile de fusel (d'après PATIL A.G.S. déjà cité) | |
|---|---|
| **Matière première** | **Huile de fusel** |
| Mêlasse de canne à sucre | 1-5% |
| Maïs | 4-5% |
| Blé | 2-3% |
| Pommes de terre | 5-11% |

Les utilisations de l'huile de fusel sont assez diverses et ont beaucoup évolué au cours des années. Avant les années 30, l'huile de fusel était utilisée seulement comme source d'alcools amyliques. Vers 1935, plusieurs études commencent à parler de l'utilisation de l'huile de fusel ou de ses dérivés en tant que solvants pour les peintures, les laques et la nitrocellulose (MAKINO Z. *et al.,* JP 111027; TING H.W. Research Inst.Ann. Rept. Bur. Chem. (1936), **3**, 75 ; CAVALIE H.R. *et al.*, FR988540). Néanmoins, la grande majorité des auteurs étudiaient la distillation et la purification de ce résidu de distillation.

Koslov *et al.* (Zhurnal Prikladnoi Khimii, (1954), 27, 223-225) ont utilisé l'huile de fusel comme agent de flottaison pour les minerais de cuivre et de zinc.

Gukasyan et al. (Tsvetnye Metallyst, (1979), 12, 61-62) ont utilisé l'huile de fusel à la place de la trioctyle-amine pour extraire le rhénium de ses solutions. D'autres auteurs ont fait appel à des phosphates d'huile de fusel pour extraire le thallium (III) (SRIVASTAVA T. N. et al., Ladbev Part A: Physical Sciences, (1971), 9, (34), 178-182), le titane (IV) (HASAN S. H. et al., Asian Journal of Chemistry, (1993), 5(2), 266-277) ou le zirconium (IV) (HASAN S.H. et al., Acta Chimica Hungaria,(1990), 127(2), 235-245).

Les alcools amyliques sont plus liposolubles que les alcools propyliques et butyliques, donc l'huile de fusel a vite trouvé sa place en tant qu'additif pour les produits pétroliers et les fluides hydrauliques. Souvent, il a été utilisé dans des mélanges de carburants pour des moteurs diesel à usage agricole (GORMAN J. W., US4585461 ; ZHANG G. M. CN 1068844; KARAOSMANOGLU F. et al., Energy Sources (1997), 19(6), 567-577).

Les esters de l'huile de fusel peuvent être industriellement utilisés comme plastifiants (GHUIBA F. M. et al., Indian Journal of Technology, (1985), 23(8), 309-311), lubrifiants (OZGULSUN A. et al., Journal of the American Oil Chemists' Society, (2000), 77(1), 105-109), agents d'extraction, arômes (WELSCH F. W. et al., Journal of Food Science (1989), 54(6), 1565-1568; YOSHIDA N. JP 01030647 ; ADNAN A. et al., Pakistan Journal of Scientific and Industrial Research (1994), 37(11), 449-452) ou émulsifiants (LOU Y., CN 1053085).

Dernièrement, plusieurs chercheurs russes ont étudié l'utilisation des acétates dérivés de l'huile de fusel d'amidon comme solvant pour les revêtements industriels ou dissolvant pour les vernis à ongles (RU 2 174 974; KORYSTIN S. I. et al., Tekhnika Mashinostroenyia (2002), 6 98-104 ; RU 2 194 492).

Aucun de ces documents ne divulgue une utilisation de solvant d'origine végétale comme solvant pour la préparation de vernis à ongles.

Or, les inventeurs ont découvert qu'un solvant d'origine végétale, l'huile de fusel estérifiée, peut être utilisé comme solvant dans la préparation de vernis, permet d'obtenir des vernis faciles à appliquer, dont le temps de séchage est de l'ordre de 2 à 4 minutes et présentant une bonne élasticité.

La présente invention a donc pour objet une composition de vernis à usage cosmétique ou pharmaceutique caractérisée en ce qu'elle comprend à titre de solvant un ou plusieurs dérivé(s) d'huile de fusel, éventuellement en mélange avec d'autres solvants d'origine naturelle, le dérivé d'huile de fusel consistant en un mélange d'acétates dont la composition est:

- 50 à 90%, avantageusement 50 à 80%, d'acétate d'isoamyle,
- 0 à 20%, avantageusement 5 à 15%, d'un mélange d'acétates de butyle et d'isobutyle,
- 0 à 20%, avantageusement 5 à 10%, d'un mélange d'acétates de propyle et d'isopropyle,
- 0 à 20%, avantageusement 5 à 15%, d'acétate d'éthyle,
- 0 à 5%, avantageusement moins de 1 % d'eau.

Selon la présente invention, l'huile de fusel utilisée est un mélange d'alcools en $C_1$-$C_5$, de préférence $C_2$-$C_5$, contenant:

- 0 à 95%, avantageusement 30 à 90%, encore plus avantageusement 50 à 80%, de 3-méthyl-1-butanol,
- 0,5 à 20%, avantageusement 5 à 15%, d'un mélange de 1-propanol et de 2-propanol et
- 2 à 63%, avantageusement 10 à 20%, d'un mélange de 1-butanol et de 2-méthyl-1-propanol.

ou contenant:

- 0 à 100%, avantageusement 30 à 90%, encore plus avantageusement 60 à 80%, de 3-méthyl-1-butanol,
- 0 à 50%, avantageusement 5 à 20%, d'un mélange de 1-butanol et de 2-méthyl-1-propanol, et
- 0 à 20% d'un mélange de 1-propanol et de 2-propanol,
- 0 à 20% d'éthanol, et
- 0 à 20%, avantageusement 0 à 5%, encore plus avantageusement moins de 1% d'eau.

Dans le cadre de l'invention, il est possible de réaliser, à partir de l'huile de fusel, des acétates par estérification, notamment les acétates choisis dans le groupe comprenant les acétates d'isoamyle, de butyle, d'isobutyle, de propyle, d'isopropyle et d'éthyle,

Si une distillation partielle ou avantageusement aucune distillation n'est réalisée après réaction, l'invention permet de former à partir de l'huile de fusel:

- un mélange d'acétates dans le cas d'une estérification, dont la composition est:

  • 50 à 90%, avantageusement 50 à 80% d'acétate d'isoamyle,
  • 0 à 20%, avantageusement 5 à 15%, d'un mélange d'acétates de butyle et d'isobutyle,
  • 0 à 20%, avantageusement 5 à 10%, d'un mélange d'acétates de propyle et d'isopropyle,
  • 0 à 20%, avantageusement 5 à 15%, d'acétate d'éthyle,
  • 0 à 5%, avantageusement moins de 1% d'eau..

Dans le cadre de la présente invention, chacune des molécules peut être utilisée en mélange avec d'autres solvants d'origine naturelle.

Dans le cadre de la présente invention, l'huile de fusel est obtenue à partir d'au moins un végétal choisi dans le groupe comprenant la mélasse de betteraves ou de canne à sucre, la pomme de terre, les céréales, la patate douce, les fruits et les déchets de ces végétaux.

L'estérification de l'huile de fusel peut être réalisée par toute méthode connue de l'homme du métier, notamment (1) par l'estérification de Fischer, réaction entre un alcool et un acide carboxylique, en présence d'un acide minéral, (2) par réaction entre un alcool et un acide carboxylique, en présence d'une résine échangeuse d'ions, (3) par réaction entre un alcool et un anhydride acide ou (4) par réaction entre un alcool et un chlorure acide.

Avantageusement, l'estérification est réalisée par réaction avec l'acide acétique en présence d'un catalyseur acide comme l'acide sulfurique ou l'acide chlorhydrique, ou en présence d'une résine échangeuse d'ions acides mise en oeuvre en batch ou en colonne simple.

A titre d'exemple de résine avantageusement utilisée selon l'invention, on peut citer les résines Dowex DR-2030, Lewatit® de Bayer, CT de Purolite ou Amberlyst® de Rohm et Haas, de préférence l'Amberlyst® 15 wet.

Les compositions de vernis selon l'invention comprennent en outre au moins une résine polyester et un agent filmogène soluble dans le solvant dérivé de l'huile de fusel, ledit agent filmogène étant avantageusement de la nitrocellulose ou un de ses dérivés, en particulier un collodion.

Conformément à l'invention, le solvant ou le mélange de solvants dérivés de l'huile de fusel représente avantageusement de 55 à 90% en poids de la composition, avantageusement de 60 à 80%, encore plus avantageusement de 65 à 75%.

Les compositions de vernis selon l'invention peuvent en outre comprendre un ou plusieurs adjuvants usuellement utilisé(s) dans le domaine cosmétique ou pharmaceutique choisi(s) dans le groupe comprenant les plastifiants, les diluants, les colorants, les pigments organiques et inorganiques, les agents thixotropiques, les écrans solaires de type UVA et UVB, les dispersants, les agents mouillants, les agents de matité, les agents d'adhésion, les agents d'enduction, les agents rhéologiques, les conservateurs, les antioxydants, les épaississants, les durcisseurs et les agents propénétrants.

Les vernis selon l'invention peuvent également comprendre au moins un principe actif à usage cosmétique ou thérapeutique, choisi dans le groupe comprenant les agents antimycosiques, les agents coricides, les agents vermicides, les agents virucides, les antibiotiques, les agents antibactériens, les agents anti-inflammatoires stéroïdiens ou non stéroïdiens, les agents antiparasitaires, les agents antiviraux et les agents immunosuppresseurs. Les concentrations en principe actif sont avantageusement comprises entre 0,001 et 10% en poids par rapport au poids total du vernis.

Les vernis selon l'invention sont préparés par des méthodes usuelles dans le domaine.

Les vernis selon l'invention peuvent être utilisés comme revêtement pour ongles, notamment comme vernis à ongles à usage cosmétique ou comme vernis à ongles à usage pharmaceutique, notamment dans les traitements des maladies dermatologiques suivantes: onychomycose, chloronychie, paronychies, érysipéloïde, onychorexie, gonococcie, granulome des piscines, larva migrans, lèpre, nodule d'Orf, nodule des trayeurs, panaris herpétique, périonyxie bactérienne aigue, périonyxie chronique, sporotrichose, syphilis, tuberculosis verrucosa cutis, tularémie, tungiase, verrues péri- et sous-unguéales, zona, les maladies dermatologiques ayant une incidence sur les ongles comme le psoriasis, psoriasis pustuleux, alopécie aerata, parakératose pustuleuse, dermatose de contact, syndrome de Reiter, parakératose pustuleurs, dermatite acrale psoriasiforme, lichen plan, atrophie idiopathique des ongles, lichen nitidus, lichen striatus, naevus épidermique verruqueux inflammatoire linéaire (NEVIL), pelade, pemphigus, pemphigode bulleuse, épidermolyse bulleuse acquise, maladie de Darier, pityriasis rubra pilaire, kératodermie palmo-plantaire, eczéma de contact, érythème polymorphe, gale, syndrome de Bazex, sclérodermie systémique, lupus, érythémateux systémique, lupus érythémeux chronique et dermatomyosite.

La présente invention a également pour objet l'utilisation d'un dérivé d'huile de fusel consistant en un mélange d'acétates dont la composition est:

- 50 à 90%, avantageusement 50 à 80%, d'acétate d'isoamyle,
- 0 à 20%, avantageusement 5 à 15%, d'un mélange d'acétates de butyle et d'isobutyle,
- 0 à 20%, avantageusement 5 à 10%, d'un mélange d'acétates de propyle et d'isopropyle,
- 0 à 20%, avantageusement 5 à 15%, d'acétate d'éthyle,
- 0 à 5%, avantageusement moins de 1% d'eau,

comme solvant dans une composition de vernis à usage cosmétique ou pharmaceutique.

Les exemples qui suivent illustrent l'invention. L'exemple 1 illustre la composition des différentes huiles de fusel en fonction de leur origine.

L'exemple 2 illustre la préparation de l'acétate d'isoamyle à partir de l'huile de fusel.

L'exemple 3 illustre une formulation de vernis de référence dans laquelle les solvants sont l'acétate d'éthyle et l'acétate de butyle.

L'exemple 4 illustre une formulation de vernis dont le ou les solvants(s) sont exclusivement d'origine naturelle et issus de l'huile de fusel estérifiée.

Les différents vernis sont préparés par des techniques classiquement utilisées dans le domaine.

**Exemple 1: Identification des composants de l'huile de fusel.**

Les compositions de trois huiles de fusel ont été déterminées par chromatographie en phase gazeuse selon les techniques connues de l'homme du métier et sont données au tableau 4 qui suit.

Tableau 4:

| Compositions des huiles de fusel (% en poids) | | | |
|---|---|---|---|
| | **Huile de fusel maïs** | **Huile de fusel betterave** | **Huile de fusel blé** |
| **EtOH** | 1,69 | 11,32 | 17,65 |
| **PrOH** | 0,03 | 0,08 | 0,09 |
| **iso-PrOH** | - | 0,05 | - |
| **BuOH** | 0,14 | 0,06 | 0,15 |
| **iso-BuOH** | 3,43 | 0,25 | 0,69 |
| **2-BuOH** | - | - | - |
| **iso-AmOH** | 74,84 | 75,73 | 57,62 |
| **Total*(%)** | **80,13** | **87,49** | **76,2** |
| * Le reste de l'huile de fusel étant essentiellement de l'eau. | | | |

**Exemple 2: Synthèse de l'acétate d'isoamyle par estérification de l'huile de fusel.**

L'huile de fusel est estérifiée par l'acide acétique (quantités stoechiométriques), en présence d'une résine échangeuse d'ions fortement acide Amberlyst® 15 (2% par rapport à la masse totale). Le mélange est chauffé à 70°C pendant 2h pour former un mélange d'acétates. La résine est filtrée pour être régénérée et le milieu réactionnel est distillé pour obtenir l'acétate d'isoamyle (P.E.: 131°C) sous forme de liquide incolore à l'odeur fruitée.

**Exemple 3: Formulation de vernis à ongles incolore (vernis de référence).**

| Fonction | Composition de référence (incolore 056) | |
|---|---|---|
| Solvants | Acétate d'éthyle | 51% |
| | Acétate butyle | 21,2% |
| Filmogène | Nitrocellulose E27 (30% IPA*) | 12,8% |
| Plastifiant | Acétyl tributyl citrate | 6% |
| Résine | Résine polyester | 9% |
| Total | | 100% |
| * IPA= Alcool isopropylique | | |

**Exemple 4: Formulation de vernis à ongles incolore.**

| Fonction | Composition nouveaux solvants | |
|---|---|---|
| Solvants | Acétate de fusel (mélange acétates en $C_2$-$C_5$) | 72,2% |

(suite)

| Fonction | Composition nouveaux solvants | |
|---|---|---|
| Filmogène | Nitrocellulose E27 (30% IPA) | 12,8% |
| Plastifiant | Acétyl tributyl citrate | 6% |
| Résine | Résine polyester | 9% |
| Total | | 100% |

*Evaluation de la facilité d'application des films.*

La référence 056 et les formulations selon l'invention ont été appliquées grâce à un applicateur Touzart-Matignon, sur des cartes de contraste, en films humides de 150 μm d'épaisseur.

*Temps de séchage.*

Les mesures du temps de séchage des formulations citées dans les exemples ont été effectuées avec un appareil de temps de séchage, sur un film de 100 μm, à une température constante de 20°C.

L'ensemble des films présente un temps de séchage voisin de celui du temps de référence, qui est de 3 minutes.

*Evaluation des émissions de COV.*

Les paramètres pris en compte pour le calcul des émissions de COV sont les suivants:
- durée d'exposition, c'est-à-dire le temps nécessaire pour réaliser le mélange des différents composants du vernis: 3h,
- nombre de formulations réalisées en 1 année: 300,
- température ambiante moyenne: 20°C,
- vitesse de l'air au-dessus de la cuve: 0,05 m/s,
- diamètre de la cuve: 1,80 m, qui correspond à une cuve de 500 1.

| Formulation | Emission de COV (kg/an) | Réduction des émissions par rapport à la référence |
|---|---|---|
| Exemple 5 (vernis de référence) acétate d'éthyle acétate de butyle | 1262,8 | 0% |
| Exemple 6 Acétates de fusel (mélange acétates en $C_2$-$C_5$) | 406,9 | - 68% |

L'ensemble des formulations selon l'invention présente une émission de COV réduite d'au moins 50% par rapport à la formulation de référence.

**Revendications**

1. Composition de vernis à usage cosmétique ou pharmaceutique, **caractérisée en ce qu'**elle comprend à titre de solvant un ou plusieurs dérivé(s) d'huile de fusel, éventuellement en mélange avec d'autres solvants d'origine naturelle, le dérivé d'huile de fusel consistant en un mélange d'acétates dont la composition est: est:

   - 50 à 90%, avantageusement 50 à 80% d'acétate d'isoamyle,
   - 0 à 20%, avantageusement 5 à 15% d'un mélange d'acétates de butyle et d'isobutyle,
   - 0 à 20%, avantageusement 5 à 10%, d'un mélange d'acétates de propyle et d'isopropyle,
   - 0 à 20%, avantageusement 5 à 15%, d'acétate d'éthyle,
   - 0 à 5%, avantageusement moins de 1% d'eau.

2. Composition de vernis selon la revendication 1, **caractérisée en ce que** l'huile de fusel est obtenue à partir d'au moins un végétal choisi dans le groupe comprenant la mélasse de betteraves ou de canne à sucre, la pomme de terre, les céréales, la patate douce, les fruits et les déchets de ces végétaux.

**3.** Composition de vernis selon l'une quelconque des revendications 1 et 2, **caractérisée en ce qu'**elle comprend en outre au moins un polymère filmogène soluble dans le solvant dérivé de l'huile de fusel, avantageusement de la nitrocellulose ou un de ses dérivés, en particulier un collodion, et éventuellement au moins une résine polyester.

**4.** Composition de vernis selon l'une quelconque des revendications 1 à 3, **caractérisée en ce que** le solvant ou le mélange de solvants représente 55 à 90 % en poids de la composition, avantageusement de 60 à 80 %, encore plus avantageusement de 65 à 75 %.

**5.** Composition de vernis selon l'une quelconque des revendications 1 à 4, **caractérisée en ce qu'**elle comprend en outre un ou plusieurs adjuvants choisis dans le groupe comprenant les plastifiants, les diluants, les colorants, les pigments organiques et inorganiques, les agents thixotropiques, les écrans solaires de type UVA et UVB, les dispersants, les agents mouillants, les agents de matité, les agents d'adhésion, les agents d'enduction, les agents rhéologiques, les conservateurs, les antioxydants, les épaississants, les durcisseurs et les agents propénétrants.

**6.** Composition de vernis selon l'une quelconque des revendications 1 à 5, **caractérisée en ce qu'**elle contient en outre au moins un composé choisi dans le groupe comprenant les agents antimycosiques, les agents coricides, les agents virucides, les agents vermicides, les antibiotiques, les agents antibactériens, les agents anti-inflammatoires, les stéroïdiens ou non stéroïdiens, les agents antiparasitaires, les agents antiviraux et les agents immunosuppresseurs.

**7.** Utilisation d'un dérivé d'huile de fusel consistant en un mélange d'acétates dont la composition est:

- 50 à 90%, avantageusement 50 à 80% d'acétate d'isoamyle,
- 0 à 20%, avantageusement 5 à 15% d'un mélange d'acétates de butyle et d'isobutyle,
- 0 à 20%, avantageusement 5 à 10%, d'un mélange d'acétates de propyle et d'isopropyle,
- 0 à 20%, avantageusement 5 à 15%, d'acétate d'éthyle,
- 0 à 5%, avantageusement moins de 1% d'eau,

comme solvant dans une composition de vernis à usage cosmétique ou pharmaceutique.

**Claims**

**1.** Varnish composition for cosmetic or pharmaceutical use, **characterised in that** it comprises, as a solvent, one or a plurality of fusel oil derivatives, optionally mixed with further solvents of natural origin, the fusel oil derivative consisting of a mixture of acetates having the following composition:

- 50 to 90%, advantageously 50 to 80% of isoamyl acetate,
- 0 to 20%, advantageously 5 to 15%, of a butyl and isobutyl acetate mixture,
- 0 to 20%, advantageously 5 to 10%, of a propyl and isopropyl acetate mixture,
- 0 to 20%, advantageously 5 to 15%, of ethyl acetate,
- 0 to 5%, advantageously less than 1% of water.

**2.** Varnish composition according to claim 1, **characterised in that** the fusel oil is obtained from at least one plant selected from the group comprising beet or sugar cane molasses, potato, cereals, sweet potato, fruit and scraps of said plants.

**3.** Varnish composition according to any of claims 1 and 2, **characterised in that** it further comprises at least one film-forming polymer soluble in the solvent derived from fusel oil, advantageously nitrocellulose or a derivative thereof, particularly a collodion, and optionally at least one polyester resin.

**4.** Varnish composition according to any of claims 1 to 3, **characterised in that** the solvent or solvent mixture represents 55 to 90% by weight of the composition, advantageously 60 to 80%, more advantageously 65 to 75%.

**5.** Varnish composition according to any of claims 1 to 4, **characterised in that** it further comprises one or a plurality of adjuvants selected in the group comprising plasticisers, diluents, colorants, organic and inorganic pigments, thixotropic agents, UVA and UVB sun screens, dispersants, wetting agents, matting agents, adhesive agents, coating agents, rheological agents, preservatives, antioxidants, thickeners, hardenings agents and pro-penetrating agents.

6. Varnish composition according to any of claims 1 to 5, **characterised in that** it further contains at least one compound selected from the group comprising antifungal agents, corn-removing agents, virucidal agents, vermicidal agents, antibiotics, antibacterial agents, steroidal or non-steroidal anti-inflammatory agents, antiparasitic agents, antiviral agents and immunosuppressant agents.

7. Use of a fusel oil derivative consisting of an acetate mixture having the following composition:

- 50 to 90%, advantageously 50 to 80% of isoamyl acetate,
- 0 to 20%, advantageously 5 to 15%, of a butyl and isobutyl acetate mixture,
- 0 to 20%, advantageously 5 to 10%, of a propyl and isopropyl acetate mixture,
- 0 to 20%, advantageously 5 to 15%, of ethyl acetate,
- 0 to 5%, advantageously less than 1% of water, as a solvent in a varnish composition for cosmetic or pharmaceutical use.

**Patentansprüche**

1. Lackzusammensetzung zur kosmetischen oder pharmazeutischen Verwendung, **dadurch gekennzeichnet, dass** sie als Lösungsmittel ein oder mehrere Fuselölderivat(e), gegebenenfalls in Mischung mit anderen Lösungsmitteln natürlichen Ursprungs, umfasst, wobei das Fuselölderivat aus einem Acetatgemisch besteht, welches zusammengesetzt ist aus:

- 50 bis 90%, vorzugsweise 50 bis 80%, Isoamylacetat;
- 0 bis 20%, vorzugsweise 5 bis 15%, eines Gemischs von Butylacetat und Isobutylacetat;
- 0 bis 20%, vorzugsweise 5 bis 10%, eines Gemischs von Propylacetat und Isopropylacetat;
- 0 bis 20%, vorzugsweise 5 bis 15% Ethylacetat
- 0 bis 5%, vorzugweise weniger als 1%, Wasser.

2. Lackzusammensetzung nach Anspruch 1, **dadurch gekennzeichnet, dass** das Fuselöl aus mindestens einer Pflanze, ausgewählt aus der Gruppe umfassend Rüben- oder Zuckerrohrmelasse, Kartoffel, Getreide, Süßkartoffel, Früchte und Abfälle dieser Pflanzen, erhalten wird.

3. Lackzusammensetzung nach einem der Ansprüche 1 und 2, **dadurch gekennzeichnet, dass** sie ferner mindestens ein filmbildendes Polymer, das in dem Fuselölderivat-Lösungsmittel löslich ist, vorzugsweise Nitrocellulose oder eines ihrer Derivate, insbesondere ein Kollodium, und eventuell mindestens ein Polyesterharz umfasst.

4. Lackzusammensetzung nach einem der Ansprüche 1 bis 3, **dadurch gekennzeichnet, dass** das Lösungsmittel oder Lösungsmittelgemisch 55 bis 90 Gew.-% der Zusammensetzung, vorzugsweise 60 bis 80%, noch mehr bevorzugt 65 bis 75% darstellt.

5. Lackzusammensetzung nach einem der Ansprüche 1 bis 4, **dadurch gekennzeichnet, dass** sie ferner ein oder mehrere Zusatz, ausgewählt aus der Gruppe umfassend Weichmacher, Verdünnungsmittel, Färbemittel, organische und anorganische Pigmente, thixotrope Mittel, Sonnenschutzmittel vom UVA- und UVB-Typ, Dispergiermittel, Befeuchtungsmittel, Mattierungsmittel, Haftmittel, Überzugsmittel, Rheologiemittel, Konservierungsmittel, Antioxidantien, Verdickungsmittel, Härtungsmittel und das Eindringen fördernde Mittel, umfasst.

6. Lackzusammensetzung nach einem der Ansprüche 1 bis 5, **dadurch gekennzeichnet, dass** sie ferner mindestens eine Verbindung, ausgewählt aus der Gruppe umfassend Antimykotika, Mittel gegen Hornhaut, Antivirusmittel, Wurmmittel, Antibiotika, antibakterielle Mittel, entzündungshemmende Mittel, Steroide oder Nicht-Steroide, Antiparasitika, antivirale Mittel und Immunsuppressiva, enthält.

7. Verwendung eines Fuselölderivats, das aus einem Acetatgemisch besteht, welches zusammengesetzt ist aus:

- 50 bis 90%, vorzugsweise 50 bis 80%, Isoamylacetat;

- 0 bis 20%, vorzugsweise 5 bis 15%, eines Gemischs von Butylacetat und Isobutylacetat;
- 0 bis 20%, vorzugsweise 5 bis 10%, eines Gemischs von Propylacetat und Isopropylacetat;
- 0 bis 20%, vorzugsweise 5 bis 15%, Ethylacetat;
- 0 bis 5%, vorzugsweise weniger als 1%, Wasser;

als Lösungsmittel in einer Lackzusammensetzung zur kosmetischen oder pharmazeutischen Verwendung.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 1390184 A
- FR 1366742 A
- JP 111027 A
- FR 988540
- US 4585461 A
- JP 01030647 A
- RU 2174974
- RU 2194492

### Littérature non-brevet citée dans la description

- **PATIL A.G.S.M et al.** *International Sugar Journal,* 2002, vol. 104, 51-5456-58
- **SHORUIGIN, P. Pet.** *Ber.,* 1933, vol. 66B, 1087-1093
- **SHORUIGIN, PP et al.** *Zhurnal Obshchei Khimii,* 1934, vol. 4, 372-394
- **KUCUK Z. et al.** *Turkish Journal of Chemistry,* 1998, vol. 22 (3), 289-300
- **KHEDR, M.A. et al.** *Pakistan Journal of Scientific and Industrial Research,* 1994, vol. 37 (11), 488-490
- *Zhurnal Prikladnoi Khimii,* 1954, vol. 27, 223-225
- **Gukasyan et al.** *Tsvetnye Metallyst,* 1979, vol. 12, 61-62
- **SRIVASTAVA T. N. et al.** *Ladbev Part A: Physical Sciences,* 1971, vol. 9 (34), 178-182
- **HASAN S. H.** *Asian Journal of Chemistry,* 1993, vol. 5 (2), 266-277
- **HASAN S.H. et al.** *Acta Chimica Hungaria,* 1990, vol. 127 (2), 235-245
- **KARAOSMANOGLU F. et al.** *Energy Sources,* 1997, vol. 19 (6), 567-577
- **GHUIBA F. M. et al.** *Indian Journal of Technology,* 1985, vol. 23 (8), 309-311
- **OZGULSUN A. et al.** *Journal of the American Oil Chemists' Society,* 2000, vol. 77 (1), 105-109
- **WELSCH F. W. et al.** *Journal of Food Science,* 1989, vol. 54 (6), 1565-1568
- **ADNAN A. et al.** *Pakistan Journal of Scientific and Industrial Research,* 1994, vol. 37 (11), 449-452
- **KORYSTIN S. I. et al.** *Tekhnika Mashinostroenyia,* 2002, vol. 6, 98-104